# FASCICULE DE BREVET EUROPEEN

(11) **EP 1 423 057 B1**
(45) Date de publication et mention de la délivrance du brevet: **06.12.2006**
(21) Numéro de dépôt: 02787163.1
(22) Date de dépôt: 17.07.2002
(51) Int. Cl.: A61B 17/80

(54) **PLAQUE D'OSTÉOSYNTHÈSE DE L'EXTRÉMITÉ SUPÉRIEURE DE L'HUMÉRUS**
OSTEOSYNTHESE-PLATTE FÜR DAS OBERE ENDE DES ARMKNOCHENS
OSTEOSYNTHESIS PLATE FOR THE UPPER END OF THE ARM BONE

(30) Priorité: 17.07.2001 FR 0109507
(43) Date de publication de la demande: 02.06.2004
(73) Titulaire: TORNIER, 38330 Saint-Ismier (FR)
(72) Inventeur: TORNIER, Alain, F-38330 Saint Ismier (FR)
(74) Mandataire: Myon, Gérard Jean-Pierre
(86) Numéro de dépôt international: PCT/FR2002/002545
(87) Numéro de publication internationale: WO 2003/007832

(56) Documents cités:
- EP-A- 0 723 764
- WO-A-98/09578
- US-A- 4 867 144
- US-A- 5 718 705
- US-A- 5 743 913
- PATENT ABSTRACTS OF JAPAN vol. 2000, no. 02, 29 février 2000 (2000-02-29) & JP 11 299804 A (HOMUZU GIKEN:KK), 2 novembre 1999 (1999-11-02)

## Description

La présente invention est relative à une plaque d'ostéosynthèse destinée à réunir des esquilles et des corps d'os fragmentés, appartenant à un humérus ou à tout autre os long.

On connaît d'après le brevet EP-A-0241914 des plaques de ce genre qui présentent une forme de T dont la branche transversale et la branche longitudinale sont percées de trous, de profil conique ovale. La plaque comporte, sur l'une des faces de chaque branche, des rainures dans lesquelles débouchent les trous qui sont prévus pour le passage de vis de fixation.

Ainsi la plaque est utilisée avec des vis pour os comportant un double filetage pour d'une part la fixation dans l'os et d'autre part la réception de ladite plaque par la mise en place d'un écrou, afin d'obtenir un dispositif de stabilisation par serrage.

La plaque permet de réduire les fractures du col anatomique de l'humérus, des extrémités de l'humérus situées du coté du coude, des extrémités supérieures du tibia ainsi que d'autres lésions au voisinages des bases.

La plaque d'ostéosynthèse suivant la présente invention consiste à perfectionner les plaques en forme de T afin d'améliorer leur fixation dans l'os et plus particulièrement le maintien et la fixation des tubérosités au niveau par exemple de l'extrémité supérieure d'un humérus.

La plaque d'ostéosynthèse suivant la présente invention est destiné à l'ostéosynthèse des fractures déplacées de l'extrémité supérieure de l'humérus et comporte un corps principal en forme de T dont la branche longitudinale et la branche transversale sont respectivement percées de trous pour le passage de vis d'ancrage et de fixation de ladite plaque contre le profil externe d'un os, et des pattes de fixation qui s'étendent à partir de chaque côté de la branche transversale du corps principal, lesdites pattes étant de plus faible épaisseur que ledit corps principal et percées de trous qui coopèrent avec d'autres vis d'ancrage pour compléter la fixation de fragments osseux au contact du profil externe de l'os.

La plaque d'ostéosynthèse suivant la présente invention comporte une branche transversale pourvue d'au moins une patte de fixation dirigée dans une direction sensiblement perpendiculaire à la branche longitudinale, au moins une patte dirigée suivant une direction parallèle à la branche longitudinale et au moins une patte qui est inclinée et comprise entre les deux premières.

La plaque d'ostéosynthèse suivant la présente invention comporte des pattes de fixation qui sont de petites dimensions et de faible épaisseur par rapport à celles prévues pour la branche longitudinale et la branche transversale afin d'être modelées au profil externe de l'os pour suivre parfaitement ses contours en venant en appui contre celui-ci.

La plaque d'ostéosynthèse suivant la présente invention comporte un corps principal qui présente une forme tuilée de rayon R1 dont la concavité est tournée vers l'os, afin de s'adapter au mieux au profil osseux dans le plan transversal.

La plaque d'ostéosynthèse suivant la présente invention comporte une courbure de rayon R située dans la région joignant la branche longitudinale et la branche transversale, et dont la convexité est tournée vers l'os, de manière à s'adapter au profil osseux dans la région d'application de la plaque.

La description qui va suivre en regard des dessins annexés, donnés à titre d'exemple non limitatifs, permettra de mieux comprendre l'invention, les caractéristiques qu'elle présente et les avantages qu'elle est susceptible de procurer:
Figure 1 est une vue en perspective illustrant le plaque d'ostéosynthèse suivant la présente invention.
Figure 2 est une vue montrant la mise en place sur un os de la plaque d'ostéosynthèse suivant la présente invention.
Figure 3 est une vue de face représentant la mise en place sur un os de la plaque d'ostéosynthèse suivant la présente invention.
Figure 4 est une vue de dessus illustrant la mise en place sur un os de la plaque d'ostéosynthèse suivant la présente invention.

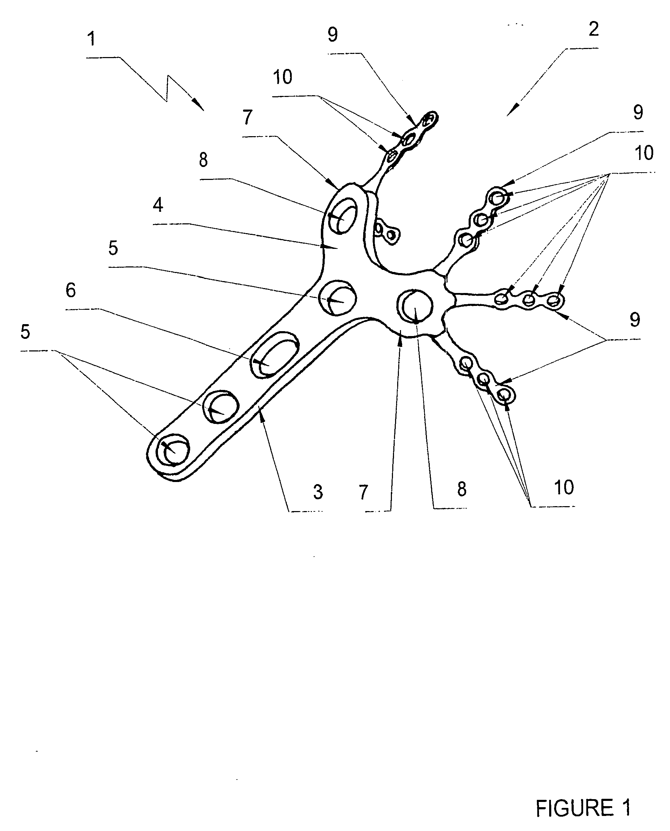

On a montré en figure 1 une plaque d'ostéosynthèse 1 comportant un corps principal 2 en forme de T délimitant une branche longitudinale 3 et une branche transversale 4.

La branche longitudinale 3 formant la partie attelle de la plaque d'ostéosynthèse 1 est percée de trous débouchants 5, entre lesquels est prévu au moins un trou 6 à profil oblong.

La branche transversale 4 formant la partie tête de la plaque d'ostéosynthèse 1 présente un profil externe légèrement courbé afin de délimiter de part et d'autre de la branche longitudinale 3 une portion arrondie 7 percée d'un trou 8.

La branche transversale 4 se prolonge au niveau de ses portions arrondies 7 d'une série de pattes de fixation 9 de plus faible épaisseur que celle du corps 2 et qui sont percées de trous 10.

Les pattes de fixation 9 s'étendent à partir du bord périphérique du corps principal 2 de la plaque d'ostéosynthèse 1.

Chaque portion arrondie 7 de la branche transversale 4 comporte au moins une patte 9 dirigée dans une direction sensiblement perpendiculaire à la branche longitudinale 3, au moins une patte 9 dirigée suivant une direction parallèle à la branche longitudinale 3 et au moins une patte 9 qui est inclinée et comprise entre les deux premières.

Les trous 10 ménagés dans les pattes 9 sont prévus de petite dimension pour recevoir des vis d'ancrage qui sont différentes de celles coopérant avec les trous 5, 6 de la branche longitudinale 3 et de la branche transversale 4.

Les pattes 9 sont de petite dimension et de plus faible épaisseur par rapport à celles prévues pour la branche longitudinale 3 et la branche transversale 4 de la plaque 1, afin d'être modelées au profil externe de l'os et suivre parfaitement ses contours en venant en appui contre celui-ci.

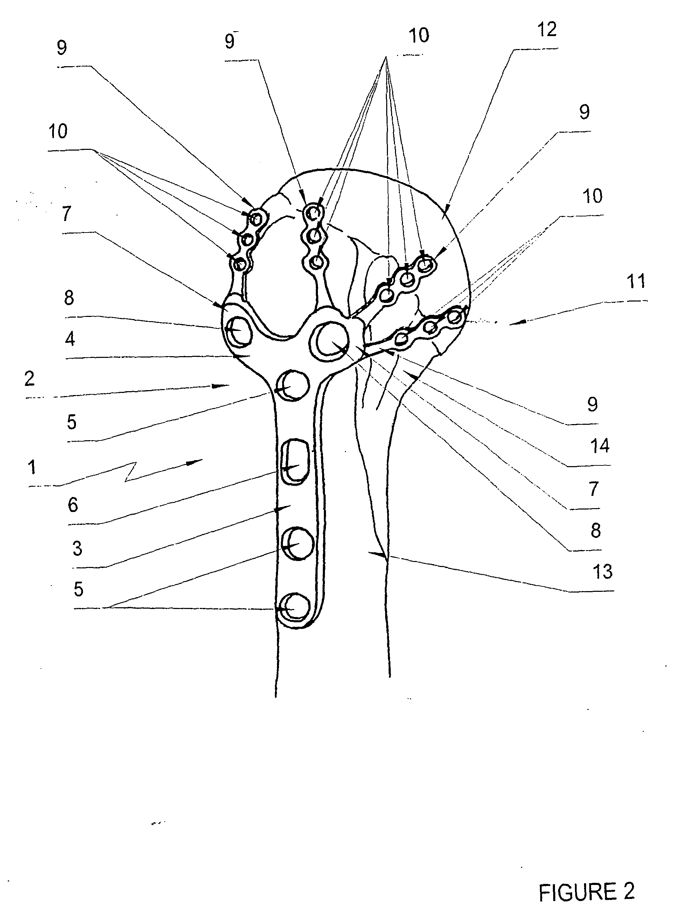

En figure 2 on a montré la plaque d'ostéosynthèse 1 en appui, par exemple, contre l'extrémité supérieure d'un humérus 11.

La mise en place de la plaque d'ostéosynthèse 1 permet, après ancrage des vis osseuses de l'attelle contre la diaphyse humérale 13, de rappeler la tête humérale 12 au contact à l'aide des vis positionnées dans les deux trous 8 de la branche transversale 4 et du trou supérieur 5 de la branche longitudinale 3.

La mise en place de la plaque d'ostéosynthèse 1 permet par ailleurs, après ancrage des vis osseuses, de maintenir et de fixer les tubérosités contre i'épiphyse de l'humérus 11.

On note que la branche longitudinale 3 de la plaque d'ostéosynthèse 1 est fixée contre la partie diaphysaire 13 de l'humérus 11, tandis que la branche transversale 4 est fixée contre la partie épiphysaire 14 de l'humérus 11, afin que les pattes 9 soient modelées autour du profil externe des tubérosité humérales avant leur fixation, par des vis d'ancrage traversant les trous 10.

On note que la direction du trou supérieur 5 de la branche longitudinale 3 et des trous 8 de la branche transversale 4 est telle qu'elle autorise le positionnement des vis dans une région proche du centre de la tête humérale 12.

Le fait que la plaque d'ostéosynthèse 1 comporte des pattes de fixation tubérositaire 9 au niveau de la branche transversale 4, permet le transfert direct des contraintes musculaires appliquées aux tubérosités au corps principal 2 en forme de T constituant ladite plaque.

Egalement l'agencement des pattes 9 les unes par rapport aux autres permet de constituer une sorte de panier qui enveloppe et maintient les tubérosités.

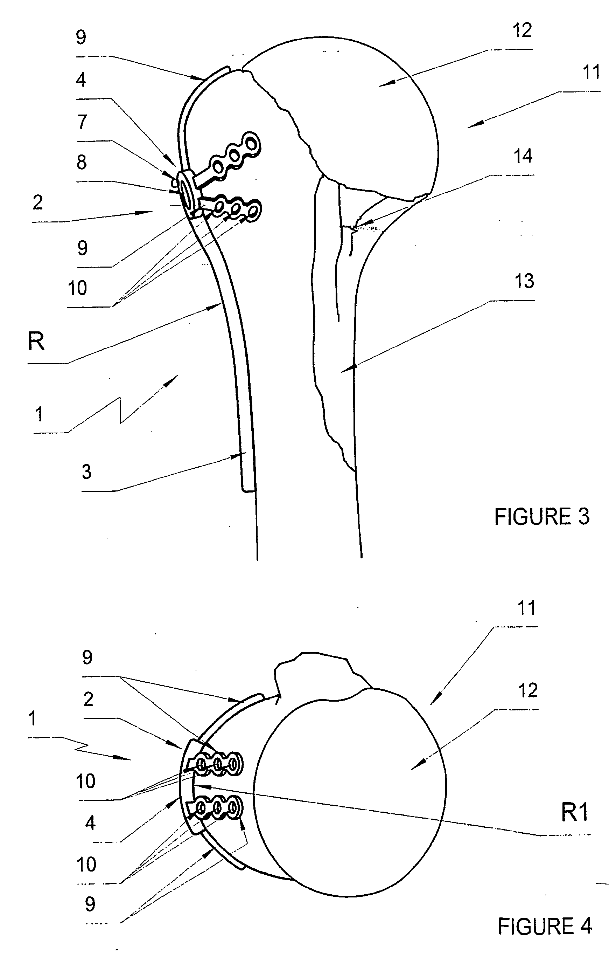

En figure 3 on a représenté la plaque 1 en vue de face qui présente une forme cintrée de rayon R située en dessous de la région joignant la branche longitudinale 3 et la branche transversale 4, dont la convexité est tournée vers l'humérus, de manière à s'adapter au profil osseux latéral dans le plan frontal.

En figure 4 on a montré l'ensemble de la plaque 1 en vue de dessus de manière à constater que le corps 2 présente une forme tuilée de rayon R1 dont la concavité est tournée vers l'humérus, afin de s'adapter au mieux au profil osseux dans le plan transversal.

On note que le corps principal 2 de la plaque d'ostéosynthèse 1 peut présenter des formes extérieures différentes sans pour cela changer l'objet de la présente invention qui consiste à disposer autour du corps 2 des pattes de fixation 9.

Il doit d'ailleurs être entendu que la description qui précède n'a été donnée qu'à titre d'exemple et qu'elle ne limite nullement le domaine de l'invention comme défini par les revendications ci-jointes dont on ne sortirait pas en remplaçant les détails d'exécution décrits par tout autre équivalent.

## Revendications

1. Plaque d'ostéosynthèse destiné à l'ostéosynthèse des fractures déplacées de l'extrémité supérieure de l'humérus comportant un corps principal (2) en forme de T dont la branche longitudinale (3) et la branche transversale (4) sont respectivement percées de trous (5, 6, 8) pour le passage de vis d'ancrage et de fixation de ladite plaque contre le profil externe d'un os, **caractérisée en ce qu'**elle comprend des pattes de fixation (9) qui s'étendent à partir de chaque côté de la branche transversale (4) du corps principal (2), lesdites pattes étant de plus faible épaisseur que ledit corps principal et percées de trous (10) qui coopérent avec d'autres vis d'ancrage pour compléter la fixation de fragments osseux au contact du profil externe de l'os.

2. Plaque d'ostéosynthèse suivant la revendication 1, **caractérisée en ce que** la branche transversale (4) comporte au moins une patte (9) dirigée dans une direction sensiblement perpendiculaire à la branche longitudinale (3), au moins une patte (9) dirigée suivant une direction parallèle à la branche longitudinale (3) et au moins une patte (9) qui est inclinée et comprise entre les deux premières.

3. Plaque d'ostéosynthèse suivant la revendication 1, **caractérisée en ce que** les pattes (9) sont de petite dimension et de faible épaisseur par rapport à celles prévues pour la branche longitudinale (3) et la branche transversale (4) afin d'être modelées au profil externe de l'os pour suivre parfaitement ses contours en venant en appui contre celui-ci.

4. Plaque d'ostéosynthèse suivant la revendication 1, **caractérisée en ce que** le corps (2) présente une forme tuilée de rayon R1 dont la concavité est tournée vers l'os, afin de s'adapter au mieux au profil osseux dans le plan transversal.

5. Plaque d'ostéosynthèse suivant la revendication 1, **caractérisée en ce qu'**elle présente une courbure de rayon R située dans la région joignant la branche longitudinale (3) et la branche transversale (4), et dont la convexité est tournée vers l'os, de manière à s'adapter au profil osseux dans la région d'application de la plaque.

## Claims

1. An osteosynthesis plate designed for the osteosynthesis of displaced fractures of the upper end of the humerus, having a main T-shaped body (2) of which the longitudinal branch (3) and the transverse branch (4) are each provided with holes (5, 6, 8) for screws to pass through for anchoring and fixing said plate against the outer profile of a bone, **characterized in that** it includes fixing straps (9) which extend from each side of the transverse branch (4) of the main body (2), said straps being thinner than said main body and being provided with holes (10) which cooperate with other anchoring screws to complete the fixing of bone fragments through contact with the outer profile of the bone.

2. An osteosynthesis plate according to Claim 1, **characterized in that** the transverse branch (4) has at least one strap (9) extending in a direction running substantially perpendicular to the longitudinal branch (3), at least one strap (9) extending in a direction running parallel to the longitudinal branch (3) and at least one strap (9) which is disposed at an angle and lies between the first two.

3. An osteosynthesis plate according to Claim 1, **characterized in that** the straps (9) are small in size and thin when compared with the longitudinal branch (3) and transverse branch (4), so as to be moulded to the outer profile of the bone and to follow its contours perfectly by coming to rest against it.

4. An osteosynthesis plate according to Claim 1, **characterized in that** the body (2) has a tiled shape of radius R1, of which the concave side is turned towards the bone so as better to adapt itself to the bone profile in the transverse plane.

5. An osteosynthesis plate according to Claim 1, **characterized in that** it has a curve of radius R located in the region joining the longitudinal branch (3) and the transverse branch (4), and of which the convex side is turned towards the bone' so as to adapt itself to the bone profile in the region of application of the plate.

## Patentansprüche

1. Osteosynthese-Platte, die für die Osteosynthese von verschobenen Brüchen des oberen Endes des Arm- bzw. Oberarmknochens bestimmt ist, umfassend einen Hauptkörper (2) in Form eines T, dessen Längsschenkel (3) und dessen Querschenkel (4) mit entsprechenden Löchern (5, 6, 8) für den Durchtritt einer Verankerungsschraube und einer Festlegung der Platte gegen das Außenprofil eines Knochens durchbrochen sind, **dadurch gekennzeichnet, daß** sie Festlegungslaschen bzw. -klauen (9) umfaßt, welche sich ausgehend von jeder Seite des querverlaufenden Schenkels (4) des Hauptkörpers (2) erstrecken, wobei die Laschen bzw. Lappen von bedeutend geringerer Dicke wie der Hauptkörper sind und mit Löchern (10) durchbrochen sind, welche mit anderen Verankerungsschrauben zusammenwirken, um die Festlegung von Knochenfragmenten in Kontakt mit dem Außenprofil des Knochens zu komplettieren bzw. zu vervollständigen.

2. Osteosynthese-Platte nach Anspruch 1, **dadurch gekennzeichnet, daß** der querverlaufende Schenkel (4) wenigstens eine Lasche (9), welche in einer Richtung im wesentlichen senkrecht zu dem Längsschenkel (3) gerichtet ist, wenigstens eine Lasche (9), die entsprechend einer Richtung parallel zu dem Längsschenkel (3) gerichtet ist, und wenigstens eine Lasche (9) umfaßt, welche geneigt ist und zwischen den zwei ersteren aufgenommen ist.

3. Osteosynthese-Platte nach Anspruch 1, **dadurch gekennzeichnet, daß** die Laschen (9) von geringer Abmessung und geringer Dicke in bezug auf jene sind, welche für den Längsschenkel (3) und den Querschenkel (4) vorgesehen sind, um entsprechend dem Außenprofil des Knochens modelliert zu werden, um perfekt seinen Konturen zu folgen, indem sie in Anlage gegen denselben gebracht sind.

4. Osteosynthese-Platte nach Anspruch 1, **dadurch gekennzeichnet, daß** der Körper (2) eine ziegelartige Form mit einem Radius R1 aufweist, deren Konkavität zu dem Knochen gerichtet ist, um sich besser an das Knochenprofil in der querverlaufenden Ebene anzupassen.

5. Osteosynthese-Platte nach Anspruch 1, **dadurch gekennzeichnet, daß** sie eine Krümmung mit einem Radius R aufweist, welche in dem Bereich angeordnet ist, welche den Längsschenkel (3) und den Querschenkel (4) verbindet, und deren Konvexität zu dem Knochen in einer derartigen Weise gerichtet ist, um sich an das Knochenprofil in dem Bereich der Aufbringung bzw. Anwendung der Platte anzupassen.
